# EUROPEAN PATENT APPLICATION

(11) **EP 2 574 350 A1**
(43) Date of publication of application: **03.04.2013**
(21) Application number: 11183233.3
(22) Date of filing: 29.09.2011
(51) Int. Cl.: A61L 26/00, A61L 27/22

(54) **Medical uses of lyophilized polymeric membranes containing blood derivatives**

(71) Applicant: Biorigen S.r.l, 16149 Genova (IT); Lorandi, Christian, 38060 Aldeno (TN) (IT); Motta, M Antonella, 38050 Tenna (TN) (IT); Migliaresi, Claudio, 38050 Tenna (IT)
(72) Inventor: Lorandi, Christian, 38060 Aldeno (TN) (IT); Motta, Antonella, 38050 Tenna (TN) (IT); Migliaresi, Claudio, 38050 Tenna (TN) (IT); Cancedda, Ranieri, 16145 Genova (IT); Mastrogiacomo, Maddalena, 16132 Genova (IT); Muraglia, Anita, 17024 Finale Ligure (GE) (IT)
(74) Representative: Capasso, Olga

(57) **Abstract**

The invention describes a freeze-dried compound composed of a polymeric matrix containing platelet rich plasma (PRP) and/or platelet lysate (PL) and/or platelet poor plasma (PPP) and/or cryoprecipitate (CRYO), and/or platelet membrane rich plasma (PMRP) which have a suitably long shelf-life and possesses, at the same time, improved characteristics of polymeric matrix re-absorption and in vivo efficacy.

The freeze-dried compound can be stored in standard ways for a suitably long period of time and be reconstituted just before the use by mean of a simple water absorption in a relatively short period of time. The reconstituted hydrogel shows physical and mechanical properties compared to those of the starting hydrogel with PRP before freeze-drying..

## Description

### BACKGROUND OF INVENTION

Valid systems to repair tissue damages and lesions, often associated with losses of substances, remain a major medical need in the clinic and surgery.

Considering the role paid by platelets and macrophages in tissue repair, the use of local applications of platelet gels has been proposed (1-15).

US 6,596,180 deals with a centrifuge system for the formation of an autologous platelet gel wherein all the blood components for the gel are derived from a patient to whom the gel is to be applied. The platelet rich plasma (PRP), formed by centrifugation of a non-coagulated whole blood and separated from the platelet poor plasma fraction, is clotted and thrombin is obtained. Thrombin is then mixed with the platelet poor plasma fraction to obtain a platelet gel.

WO 2008/004260 presents a biomembrane with angiogenic activity for implant in tissue regeneration and repair, including bone reconstruction and repair of skin and soft tissue lesions. The biomembrane is essentially constituted by a gel able to provide support and growth and/or differentiation and/or angiogenic factors for the full in vivo functionality of the cells. It contains also mesenchymal stem/precursor cells. This document also discloses an implant device for reconstructive surgery of bone tissue, of skin and soft tissue lesions which comprises the biomembrane and a method to obtain it. Use of the gel alone for tissue regeneration and the use of adhesive plasters that comprise the gel is also described. Hokugo A et al. (16) describes a method to prepare a cross-linked gelatin hydrogel containing PRP. The gel is formed by adsorption of PRP on freeze-dried cross-linked gelatine rods. This method is used for immediate or almost immediate use of the hydrogel rods. Similarly, cross-linked gelatin hydrogel microspheres and membranes containing PRP have been produced by others (17-24).

Biomembranes, or more properly artificial biomembranes to differentiate them from the biological membrane in the organism, are, in a general definition, gels in form of film or thin layers of different size and shape composed of biocompatible polymers with or without a supporting scaffold structure.

Bio-membranes containing PRP, or more in general blood derivatives, are used for tissue repair of large lesions. Such bio-membranes may also contain biologically active agents. They are used by placing them in contact with the organ to be treated and where the biologically active agent needs to be released. The bio-membranes containing PRP have proven to have a lower efficacy than that of the pure PRP gel. However, they display a much higher flexibility and better handling properties given by the physical and mechanical properties of their structure. The bio-membranes are easily handled by the doctor, suitably implanted or placed in the site of contact with the targeted organ (i.e. skin) where they remain well fixed and release the biologically active agents.

Furthermore, these gelatin bio-membranes are bio-compatible and biodegradable. However, they are cross-linked polymeric matrixes which represent a major handicap since chemical cross-linking has a potential toxic effect due to cross-linking agents (i.e. residual monomers in the matrix and/or compounds formed during the in vivo degradation). Bio-membrane hydrogels containing PRP are in general prepared and immediately implanted or used at the site of repair. In fact, the stability of the hydrogels is poor and the gels can be conserved only for a limited period of time at a low temperature (higher than 0°C, i.e. from 1°C to 5°C). Freezing the bio-membrane hydrogels containing PRP at a temperature below 0°C (the freezing temperature for this compounds often reaches -80°C) would damage irreversibly its physical structure due to creeping. In addition at these temperatures the stability of the incorporated active molecules, including PRP, is affected with increased storage time.

The gel formation by the addition of PRP as described in the Hokugo et al. (16) and other related references is not a straightforward process to be carried out in surgical operation rooms or in doctor officies.

There is therefore the need to provide bio-membrane containing PRP and/or blood derivatives and/or conditioned cell culture medium having increased stability and shelf-life while maintaining their in vivo repair properties. Indeed a product ready to use or requiring simple procedures for preparation is needed for the medical community.

Further, there is the need for a simple and cost effective method for the preparation of bio-membrane containing PRP and/or blood derivatives and/or conditioned cell culture medium having increased stability and shelf-life while maintaining their in vivo repair properties.

### DESCRIPTION OF THE INVENTION

The authors have unexpectedly found that a stable product with improved characteristics of polymeric matrix re-absorption and in vivo efficacy can be obtained by freeze-drying the polymeric based hydrogel containing PRP and/or platelet lysate (PL) and/or platelet poor plasma (PPP) and/or cryoprecipitate (CRYO) and/or platelet membrane rich plasma (PMRP).

The freeze-dried compound of the invention can be stored in standard ways (i.e. at -20°C in a standard freezer) for a suitably long period of time and be reconstituted just before the use by mean of a simple water absorption in a relatively short period of time. On the contrary, the prior art frozen gel of PRP, to be stored at -80°C to keep unmodified its biological characteristics for a sufficient shelf-life, cannot be utilized after thawing due to the gel cracking during the process.

The reconstituted hydrogel from the freeze-dried compound shows physical and mechanical properties compared to those of the starting hydrogel with PRP and/or PL and/or PPP and/or CRYO, and/or PMRP before freeze-drying, thus it can be easily handled by the doctors and remain fixed in the site of implantation or application.

It is therefore an object of the invention a lyophilized gelatin membrane containing an effective amount of at least one blood derivative selected from the group of: platelet rich plasma (PRP), platelet lysate (PL) platelet poor plasma (PPP), cryoprecipitate (CRYO), platelet membrane rich plasma (PMRP), for use as a tissue regenerating agent by topical administration.

Preferably the effective amount of the at least one blood derivative is loaded onto the membrane prior to the lyophilization.

In a preferred embodiment, the lyophilized gelatin membrane of the invention is for a fast release of the at least one blood derivative.

In a preferred embodiment, the lyophilized gelatin membrane of the invention is obtainable by:
a) dissolving a gelatin powder in an effective amount at least one blood derivative to get a solution of gelatin / at least one blood derivative;
b) gelifying said solution to get a membrane of at least one blood derivative;
c) lyophilizing said membrane.

In a preferred embodiment, the lyophilized gelatin membrane obtained by the above process is for a prolonged release of the at least one blood derivative.

Preferably the tissue is skin, derma and/or connective tissue.

More preferably, the lyophilized gelatin membrane of the invention is for use as a tissue regenerating agent is further to a necrosis or a burning event.

Still preferably, the lyophilized gelatin membrane of the invention is for use for wound or bed sores healing.

Preferably, the lyophilized gelatin membrane of the invention is for use as a tissue regenerating agent is due to a metabolic or degenerative disease.

In the present invention, the inventors have prepared the polymeric hydrogel (bio-membrane) containing products derived from the blood platelet fraction as PRP and/or PL and/or PPP and/or CRYO, and/or PMRP in two ways.

In a first method, PRP and/or PL and/or and/or PPP and/or CRYO, and/or PMRP are absorbed on a dehydrated gelatin based matrix, previously prepared by freeze-drying or heating of the gelatin based hydrogel.

Alternatively the gelatin based bio-membranes containing platelet rich plasma (PRP) and/or PL and/or PPP and/or CRYO and/or PMRP, could be prepared by dissolving the gelatin powder directly in PRP, and/or PL and/or PPP and/or CRYO, and/or PMRP. In this condition formation of the gel occurs at room temperature followed by freezing-drying and sterilization.

The first method is preferably used for immediate or almost immediate release of PRP and/or PL and/or PPP and/or CRYO, and/or PMRP. The second method is preferably used for a prolonged release of PRP and/or PL, and/or PPP and/or CRYO, and/or PMRP. According to the invention the membranes are initially frozen by mechanical refrigeration or dry ice. The freezing temperature is between -50°C and -80°C. During the primary drying phase, the pressure is lowered to the range of a few millibars. During the initial drying phase, about 95% of the water in the material is sublimated. The final pressure at the time when all water is sublimated can be as low as 0.001 mbar.

According to the invention, the freeze-dried compound containing PRP and/or platelet products and/or blood derivatives can be stored for a long period of time at a temperature from -30°C to +30°C, preferably from -25°C to +10°C, most preferably from -20°C to 0°C. According to the invention, the bio-membranes are biocompatible, biodegradable and bioreabsorbable in any ways such as erosion, melting and/or gel-sol transition, dissolution, enzymatic. The bio-membranes are composed of not cross-linked polymeric structures. As a not limitative example, bio-membranes are made of gelatin, collagen, poly lactic acid, poly gluconic acid, pectin, alginate, carragenan, chitosan, chitin, pure or composite thereof. According to the invention, the reconstituted hydrogel is absorbed in vivo in a time ranging from 6 hours to 2 months, preferably from 1 day to 1 month, most preferably from 7 days to 15 days.

In the present invention PPP means Platelet Poor Plasma, PL means Platelet Lysate, PRP means Platelet Rich Plasma, PMRP means Platelet Membrane Rich Plasma and CRYO means Cryo-precipitate. In the context of the present invention a bio-membrane is a membrane formed by natural polymers or bio-compatible polymers. A gel is a 3D solid, jelly-like material that can have properties ranging from soft and weak to hard and tough. In the present description, it is made of gelatin or other biocompatible, biodegradable polymers with water swelling properties. A film is a gel in a 2D-like form (the dimensions of two dimensions are much bigger than that of the third one, namely thickness).

The invention is now described by reference to the following non-limiting figures and examples.

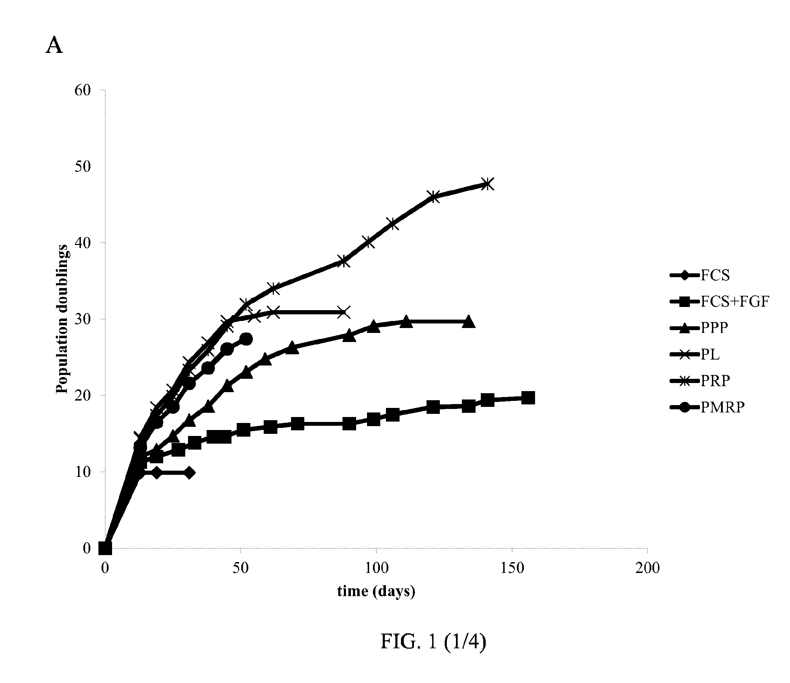

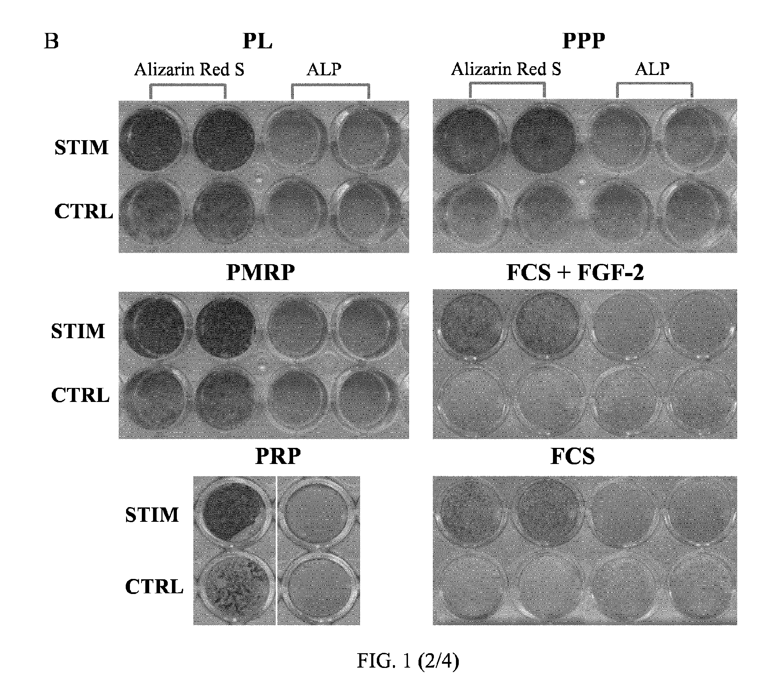

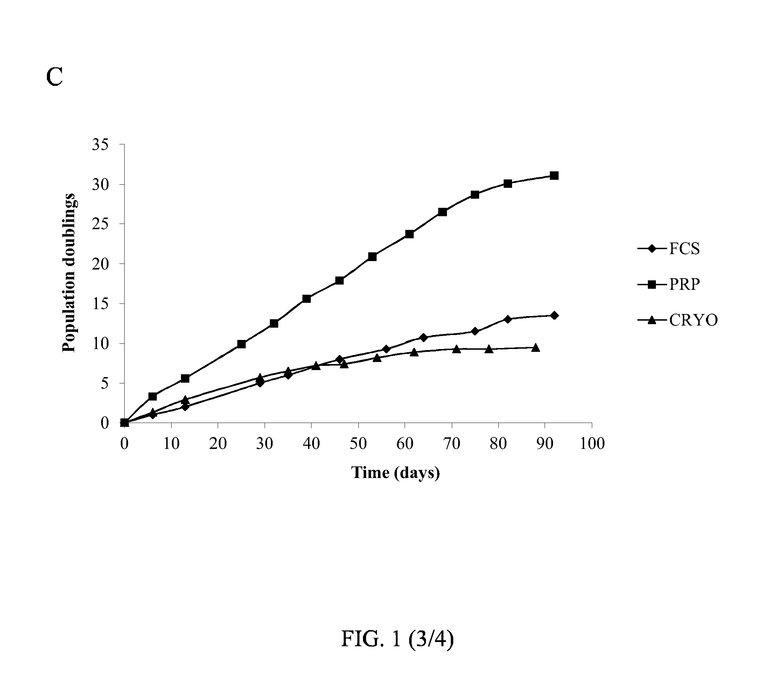

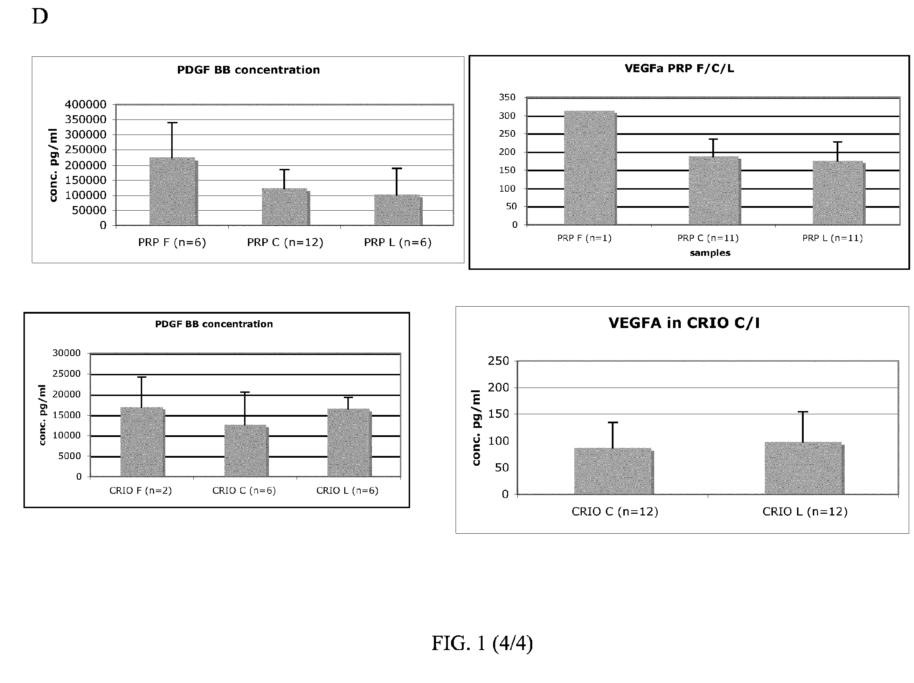

**Fig 1: A****)** Human bone marrow derived mesenchymal stem cells (hMSC) doublings at different time of culture. Cells were expanded with media supplemented with platelet products PRP, PL, PPP, PRMP at 5% concentration. Control cultures were maintained in 10% FCS or 10% FCS + 1 ng/mL FGF-2 (Fibroblast Growth Factor-2); **B)** In vitro osteogenic differentiation of hMSC expanded with different media containing 10% FCS or 10% FCS + 1 ng/ml FGF-2 or 5% PPP or 5% PL or 5% PRP or 5% PMRP. hMSC were induced with an osteogenic medium and after 12 days were stained for alkaline phosphatase (ALP), a marker of osteogenic differentiation, and Alizarin Red S to reveal calcium deposition; **C)** Proliferation rate (population doublings) of human fibroblasts expanded in different media containing 10% FCS or 5% CRYO or 5% PRP; **D)** PDGF-BB (Platelet Derived Growth Factor-BB) and VEGF-A (Vascular Endothelial Growth Factor-A) quantification in PRP/CRYO samples. The growth factors content was determined by Elisa test in fresh platelet rich plasma or cryoprecipitate samples (PRP F and CRYO F), in frozen platelet rich plasma or cryoprecipitate samples (PRP C and CRYO C) and in lyophilized platelet rich plasma or cryoprecipitate samples (PRP L and CRYO L).

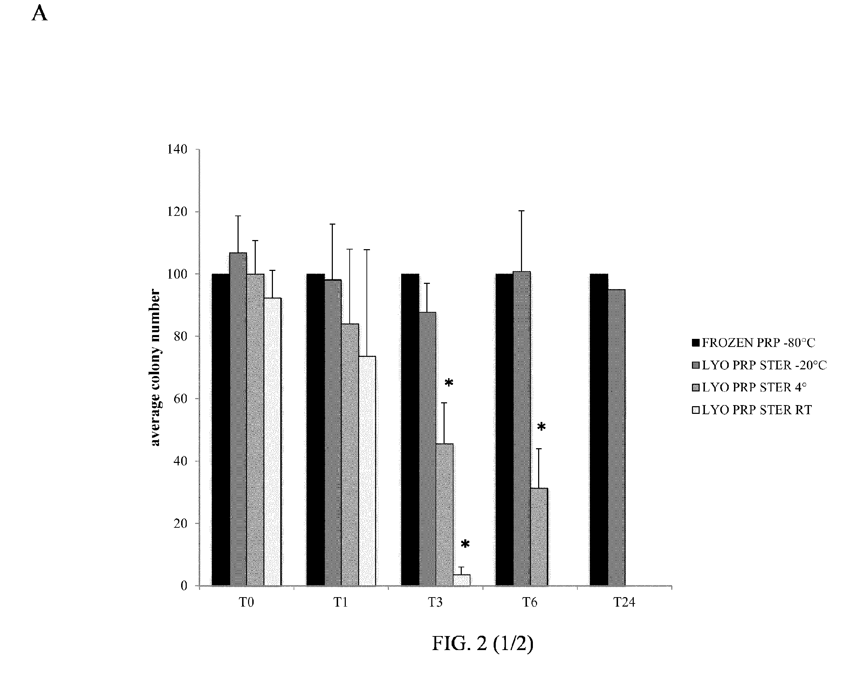

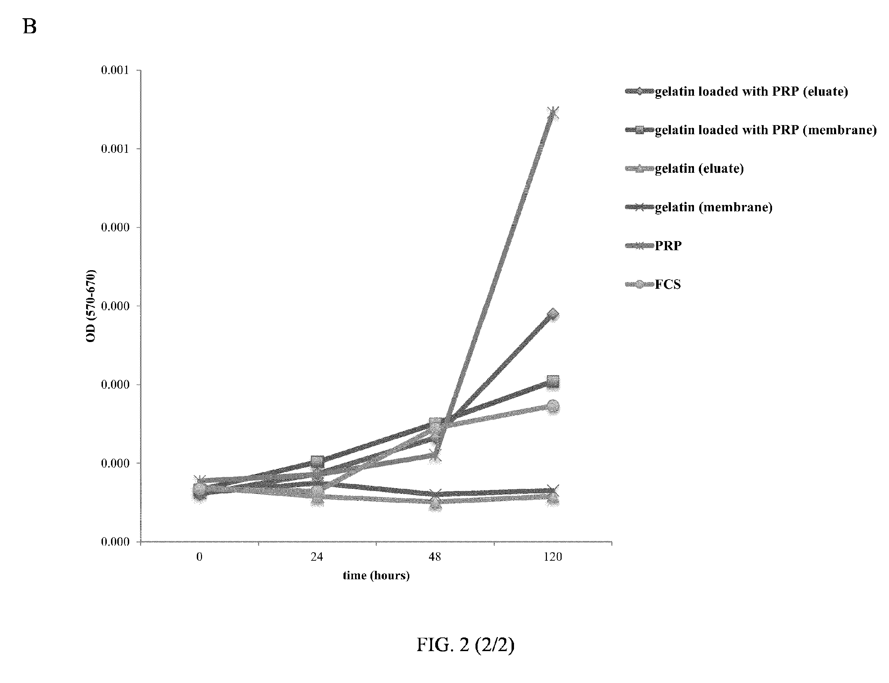

**Fig. 2: A****)** Average colony number at different times after treatment of cells with lyophilized PRP normalized to frozen PRP -80°C condition. Different aliquots of the same freeze-dried PRP preparation were stored at room temperature (RT), 4°C and -20°C. For each of these conditions, PRP preparations were evaluated for clonogenic potential immediately after preparation (T0), 1 month (T1), 3 months (T3), 6 months (T6), 24 months (T24) storage; **B)** Proliferation kinetics of hMSC cultured with gelatin membrane loaded with PRP (produced as described in example 2) assessed with the MTT assay hMSC were cultured in 10%FCS until 80% confluence, then detached and replated in a 24-well culture plate at a density of 5x10³ cells/well in triplicate. After 24 hours, the medium was removed and replaced with serum-free medium supplemented with different preparations at 5% concentration: 1) eluate derived from the melting of a gelatin membrane loaded with PRP fragment at 42°C; 2) gelatin membrane loaded with PRP as solid fragment; 3) eluate derived from the melting of a gelatin membrane fragment at 42°C; 4) gelatin membrane as solid fragment. Control cultures were performed with 10%FCS and 5% frozen PRP.

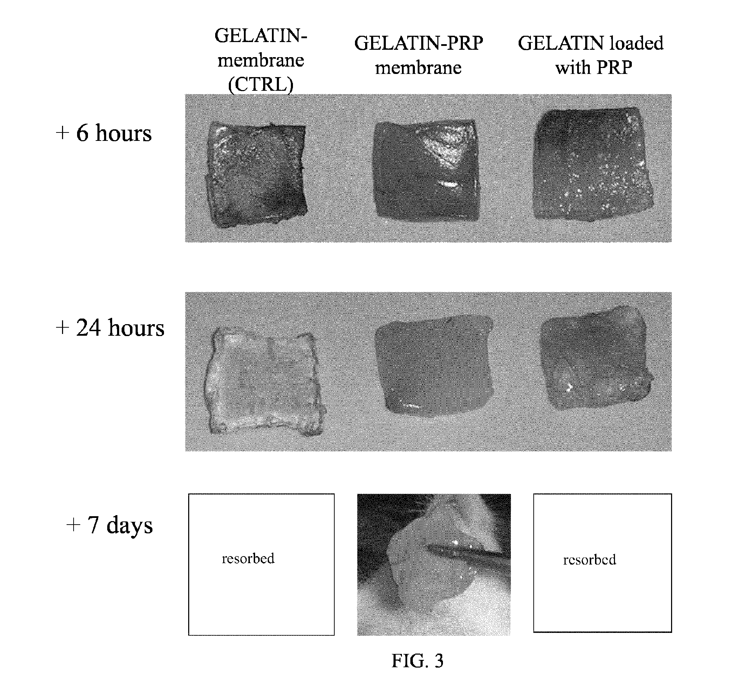

**Fig. 3****:** Macroscopic view of the PRP gelatin membranes (prepared as described in examples 2 and 3) implanted in CD1 mice and retrieved at different times.

### EXAMPLES

### Example 1 - PRP, PPP, PL, PMRP preparation

Products derived from blood platelet fractions PRP and PPP can be prepared in different ways. As a not limiting example, PRP and PPP derivatives were prepared from pooling buffy coats derived from high speed centrifuged whole blood. After the centrifugation, the plasma and buffy layer were carefully removed, transferred to a fresh tube and centrifuged at a revolution speed higher than 850 rpm in order to recover the platelets.

The upper phase, represented by the PPP, was carefully transferred to a fresh tube to derive the cryoprecipitate (CRYO). The platelet pellet was recovered and diluted with an appropriate volume of PPP in order to obtain a PRP preparation with a defined platelet concentration (from 1 x10⁶ to 10 x10⁶ platelets/µl). Cryoprecipitate is produced by slow-thawing of frozen PPP, at low temperature (4°C).

PL (platelet lysate) is obtained from a PRP preparation at a platelet concentration between 1x10⁶ and 10x10⁶/µl which has been frozen at -80°C in polypropylene tubes. The tubes are immersed into liquid nitrogen for 1 min and then immediately transferred to a 37°C water bath for 6 min. This freeze-thaw cycle is repeated three times in order to break the platelet membranes and to release their growth factor content (platelet activation). The activated PRP is centrifuged in high speed centrifuge tubes at 19000xg for 3 min to precipitate the broken platelet membranes (pellet). The supernatant represents the PL which is collected and stored at -80°C. The pellet resuspended with PPP in the same volume of the removed PL represents the PMRP which is stored at -80°C.

Each platelet product (PRP, PPP, CRYO, PL, PMRP) is then frozen at -80°C for at least overnight and then lyophilized for about 15 hours.

Reconstitution of the lyophilized platelet fraction derived products is done by adding distilled sterile water in the same amount of the lyophilized volume of the product.

### Example 2 - Preparation of lyophilized PRP gelatin membrane

The gel is formed by adsorption of PRP on a dehydrated gelatin membrane, previously prepared by freeze-drying of a gelatin based hydrogel. The hydrogel has been previously prepared by dissolving the gelatin in water. Gelatin powder (gelatin from porcine skin, Sigma G8150) is poured under stirring into water at 40-50°C. The solution is filtered at 0,22 µm to remove the suspended particles and for sterilization. The filtered solution is then poured into a suitable mold and cooled at room temperature.

100 mg lyophilized gelatin membrane is loaded with PRP drop by drop by means of a pipette about 3-4 times its volume for 2 hours at room temperature on an orbital shaker. The excess liquid is removed, the membrane is frozen at -80°C for at least 16 hours, then lyophilized for 15 hours. During the freezing-drying process to avoid membrane folding, a sterilized stainless steel grid is used to maintain the membrane flat. After freeze-drying, the lyophilized gelatin membrane loaded with PRP is sterilized by gamma radiation.

### Example 3 - Preparation of lyophilized PRP gelatin membrane

In this example gelatin powder (gelatin from porcine skin, Sigma G8150) is directly dissolved in PRP in order to obtain a gelatin-PRP membrane, then lyophilized and sterilized.

Briefly, the gelatin is dissolved in PRP at 37°C at a 15% concentration (w/v) under constant agitation for 1-2 hours. When the gelatin-PRP solution is homogeneous, it is transferred to a sterile plate and maintained for at least one hour at room temperature for the gelification. Then the gelatin-PRP membrane (i.e. from 0,5 mm to 2 cm thickness) is frozen in the plate at -80°C for at least 16 hours, lyophilized, poured in a sealant bag and sterilized by gamma radiation.

### Example 4- Biological activity of the blood platelet fraction derived products on human cells proliferation

The biological activity of the PRP, PL, PMRP and PPP on human cells proliferation was evaluated in vitro. Human MSC were obtained from bone marrow aspirate of patient undergoing orthopaedic surgery after informed consent. The nucleated cells were plated in 10% FCS containing medium for 24 hours, then the cells were transferred to a serum free medium supplemented with 5% PRP, or 5% PL, or 5% PPP or or 5% PRMP. 20 IU/ml heparin was added to the cultures containing the platelet products in order to avoid gel formation. Standard culture conditions 10% FCS and 10% FCS+FGF-2 were performed as control cultures. At 80% confluence, cells were enzymatically detached and replated for several passages in order to determine number of doublings and to establish the cell life span. At each passage, the number of doublings was calculated according to the formula: Log₂ of cells obtained/cells plated and plotted against time in culture. (Fig. 1A).

The number of cell doublings was much higher, from 3 to 5 times, with supplements of the different platelet products (in the rank PRP>PL>PMPR>PPP) than using the standard culture conditions.

The "in vitro" osteogenic potential of hMSC cultured in the presence of the different platelet products was investigated. The cells isolated from the bone marrow sample were expanded until 80% confluence. After trypsinization, passage 0 the MSC were replated in 24-well culture plates at a density of 5x10⁴ cells/well. At confluence cells were treated with an osteogenic inductive medium containing 50 µg/ml ascorbic acid, 10 mM β-glycerophosphate and 10⁻⁷ M dexamathasone. Negative control cultures were maintained in the corresponding medium without osteogenic inducers. The medium was changed three times weekly and osteogenic differentiation observed for 12 days. The osteogenic differentiation was verified by the alkaline phosphatase histochemical staining of the mineralized matrix and with the Alizarin Red S staining of the deposited calcium. Fig. 1B shows that cells expanded with PRP or PL or PPP or PRMP presented a similar osteogenic differentiation as compared to the cells grown in standard conditions, i.e FCS or FCS + FGF-2.

The proliferation of human skin fibroblasts cultured with different platelet products was also evaluated. Cells were isolated from surgical specimens which were minced and plated in medium containing 10% FCS. Skin fragments were held to the bottom of the culture dishes by sterile cover glasses until a significant cell growth was reached. After confluence, cells were detached with trypsin and replated in dishes for several passages in serum free medium containing 5% PRP or 5% CRYO in order to establish the cell life span. Control culture was maintained in medium with 10% FCS. At each passage, the number of doublings was calculated according to the formula: Log₂ of cells obtained/cells plated and plotted against time in culture. Compared to the FCS expansion condition, the proliferation rate was exalted by PRP (about 3 times higher than the standard FCS) while the CRYO supplement had similar effect to the standard culture condition (10% FCS) (Fig. 1C)

The PDGF-BB and VEGF-A content in fresh, frozen and lyophilized PRP and CRYO preparations was evaluated by Elisa test. As shown in Fig. 1D, PDGF-BB and VEGF-A concentrations did not significantly differ between the frozen and lyophilized samples showing that the freeze-drying process does not alter the concentration of the analyzed growth factors.

### Example 5 - Biological activity of the lyophilized PRP

Different aliquots of the same freeze-dried sterilized PRP preparations were stored at room temperature (RT), 4°C and -20°C. The control culture condition was represented by the frozen PRP maintained at -80°C. Three different PRP preparations were tested.

For each of the storage conditions reported above, the biological activity of the lyophilized PRP was verified immediately after production (T0) and after 1 month (T1), 3 months (T3), 6 months (T6), and 24 (T24) months of storage with the CFU-f (Colony Forming Unit-fibroblasts) clonogenic assay by hMSC. Bone marrow sample was plated at low cellular density in 10% FCS medium and after 72 hours the medium was replaced with serum-free medium containing 5% of the lyophilized PRP stored at the different temperatures. After 10-14 days of culture, colonies derived from adherent stem cells were stained with methylene blue and counted with the ImageJ software.

As shown in Fig. 2A, cultures performed with lyophilized PRP preparations stored at 4°C display a good stability after 1 month, but showed a significant decrease in colony number after 3 and 6 months of storage vanishing completely at later time points. Cultures grown with PRP preparations stored at RT showed a strong significant decrease in colony number after 3 months of storage and no more colonies are formed at 6 month RT storage.

The cell cultures treated with the lyophilized PRP preparations stored at -20°C up to 24 months showed the best performance and the colony number was comparable to the control condition (frozen PRP -80°C).

### Example 6 - Biological activity of the lyophilized gelatin PRP bio-membrane

The biological activity of the lyophilized gelatin membrane loaded with PRP (produced according to the example 2) versus the control gelatin bio-membrane was assessed in vitro. The lyophilized bio-membranes were stored at either 4°C or -20°C for 1 week before use and their activity was tested by determining the proliferation kinetics of human MSC with the MTT (3-(4,5-Dimethylthiazolil-2-yl)-2,5-diphenyltetrazolium bromide) assay. hMSC were cultured in 10% FCS until 80% confluence, then detached and replated in a 24-well culture plate at a density of 5x10³ cells/well in triplicate. After 24 hours, the medium was removed and replaced with serum-free medium supplemented with different preparations at 5% concentration: 1) eluate derived from the melting of a gelatin membrane loaded with PRP fragment at 42°C; 2) gelatin membrane loaded with PRP as solid fragment; 3) eluate derived from the melting of a gelatin membrane fragment at 42°C; 4) gelatin membrane as solid fragment. Control cultures were performed with 10%FCS and 5% frozen PRP. Growth kinetics was measured for 5 days and the absorbance readings were performed on a spectrophotometer using test and reference wavelengths of 570 and 670 nm respectively. As shown in Fig. 2B cultures supplemented with the gelatin membrane loaded with PRP, either the whole gelatin membrane or the gelatin membrane eluate, promoted cell proliferation similarly to cell proliferation in standard culture medium (10% FCS). On the contrary in cultures supplemented with membrane non loaded with PRP no cell proliferation was observed. As already shown, cells cultured in the presence of 5% PRP showed the best proliferation kinetics. This suggests a controlled release of the absorbed factors by the gelatin membranes.

### Example 7 - Chemical physical characteristics of the lyophilized gelatin PRP bio-membrane.

The chemical and physical characteristics of the lyophilized PRP preparation made according to Example 3 was assessed. The evaluations were performed on lyophilized bio-membrane immediately after production (T0) and after 1 month (T1), 3 months (T3), and 6 months (T6) of storage at -80°C.

The physical resistance (elastic modulus E) was determined on a 0,5 inch diameter gel disc by means of an Instron 4502 (Instron Italia) at a 1,3 mm/min testing speed and a temperature of 23°C and 33°C.

The melting temperature (gel-sol transition) was determined by means of Differential Scanning Calorimetry (DSC) using a Mettler DSC30 equipped with a StarE v.6 software at 1°C/min scan rate.

Results are reported in Table I.

**Table I: Melting temperature and elastic modulus of lyophilized PRP bio-membrane stored at -80°C**

| Storage (months) | Melting Temperature (°C) | E (KPa) at 23°C | E (KPa) at 33°C |
|---|---|---|---|
| 0 | 36,3 | 84,2 | 26,0 |
| 1 | 36,3 | 84,0 | 26,1 |
| 3 | 36,0 | 83,8 | 25,4 |
| 6 | 36,1 | 83,7 | 25,7 |

The results showed that the physical characteristics (melting temperature and elastic modulus) were maintained in the storage conditions up to 6 months. Moreover, the absolute values of E indicated that the lyophilized PRP bio-membrane can be easily handled during the different process steps.

### Example 8 - In vivo resorption of the lyophilized membranes

The lyophilized PRP gelatin membranes produced according to the methods reported in example 2 and example 3 were implanted (1 sample for condition) subcutaneously in six CD1 mice. Each mouse was implanted with one sample of gelatin membrane alone (CTRL), one sample of gelatin membrane loaded with PRP (prepared as described in example 2), one sample of gelatin-PRP membrane (prepared as described in example 3). The implanted samples were retrieved after 6 h, 24 h and 7 days.

The resorption of the membrane was evaluated by visual inspection, as clearly reported in the pictures of Fig. 3. The gelatin membrane alone (CTRL) was resorbed within 24 hours, the gelatin membrane loaded with PRP was resorbed within 7 days, the gelatin-PRP membrane was still present at 7 days.

As reported in the method section, the membrane prepared according to example 2 releases PRP faster than the membrane made according to example 3. Compared to the control, the erosion/resorption rate was slower. This result suggests a strengthened interaction between PRP and gelatin, thus allowing PRP to exert properly its functions in vivo.

### Example 9 - In vivo properties of lyophilized PRP membrane

The in vivo performances of the lyophilized PRP membrane made according to the Example 2 were investigated.

A PRP gelatin hydrogel, which did not undergo to the lyophilization process, was taken as the gold standard (reference hydrogel).

### In vivo resorption properties

Both the hydrogels were implanted subcutaneously in mouse as in Example 8. The mice were sacrificed after 15 days and the resorption of PRP gelatin hydrogel membranes was evaluated visually, observing if the membrane is still present and, if yes, by weighing the residual amount and normalize it to the membrane before implant. The results are reported in Table II.

**Table II: Resorption grade of PRP-gelatin hydrogel membranes.**

| | Hydrogel produced as in example 2 | Reference hydrogel |
|---|---|---|
| Resorption | 100% | 75% |

PRP-gelatin membrane according to the invention (example 2) showed a full bio-resorption in 15 days, while the reference was still present in a residual amount. The bio-resorption of the PRP-gelatin membrane according to the invention is therefore better.

### In vivo effect on necrosis and inflammation

The capability of the lyophilized gelatin membrane loaded with this conditioned medium to promote new blood vessel formation, to restore the normal perfusion levels in an ischemic damaged area and to reduce the necrotic area was tested in a rat model of ischemic full-thick skin flap elevated on the epigastric region (25).

The rat were sacrificed after 6 hours, 24 hours and 7 days. The recovery from necrosis and the presence of inflammation (biocompatibility) in the surrounding area were evaluated by means of histology. The results are reported in Table III.

**Table III: Grade of necrosis and inflammation in PRP-gelatin hydrogel membranes**

| Time | Hydrogel produced as in example 2 | | Reference hydrogel | |
|---|---|---|---|---|
| | % necrosis | Inflammation | % necrosis | Inflammation |
| 6 hours | 90 | Poor | 100 | No |
| 24 hours | 85 | Poor | 75 | No |
| 7 days | 60 | No | 50 | No |

PRP-gelatin membrane according to the invention (example 2) showed a better biological performances (less necrosis and inflammation) than those of the reference.

### In vivo effect on skin regeneration after burning

Both the hydrogels were evaluated in hairless mice with severe burning on the skin (experimental reference 26) The recovery from the burning was evaluated by determining the grade (%) of remaining burning by histological examination at 1, 2, 5 and 10 days. The remaining burning area was measured and normalized to that at time zero. The results are reported in the Table IV.

**Table IV: % burning at different time after application of PRP gelatin biomembranes**

| Days | % Burning Hydrogel produced as in example 2 | % Burning Reference hydrogel |
|---|---|---|
| 0 | 100 | 100 |
| 1 | 95 | 100 |
| 2 | 90 | 95 |
| 5 | 65 | 80 |
| 10 | 40 | 60 |

A marked improvement of burning recovery (faster healing) was found after application of the PRP gelatin bio-membrane according to the invention.

### REFERENCES

1. Rappl L.M.. Int Wound J. 2011; 8(2):187-95.
2. Akingboye AA, et al., J Extra Corpor Technol. 2010; 42(1):20-9.
3. Vogrin M, et al., Eur Surg Res. 2010; 45(2):77-85.
4. Horstmann WG, et al., Knee Surg Sports Traumatol Arthrosc. 2011; 19(1):115-21.
5. Frykberg RG, et al., Ostomy Wound Manage. 2010; 56(6):36-44.
6. Cenni E, et al., Musculoskelet Surg. 2010; 94(1):1-8
7. Kazakos K, et al., Injury. 2009; 40(8):801-5
8. Ficarelli E, et al., Dermatol Ther. 2008; 21 Suppl 1:S13-7.
9. Hom DB, Linzie BM, Huang TC. Arch Facial Plast Surg. 2007; 9(3):174-83.
10. Anzarut A, et al., Plast Reconstr Surg. 2007; 119(4):1159-66.
11. Vang SN, et al., J Extra Corpor Technol. 2007; 39(1):31-8.
12. Driver VR, et al., Ostomy Wound Manage. 2006; 52(6):68-70, 72, 74 passim.
13. Carter CA, et al., Exp Mol Pathol 2003; 74 (3):244-55.
14. Bhanot S, Alex JC. Facial Plast Surg. 2002; 18(1):27-33.
15. Whitman DH, et al., J. Oral Maxillofac. Surg. 1997; 55(11):1294-9.
16. Hokugo A, et al., Tissue Eng. 2005; 11(7-8):1224-33
17. Sawamura K, et al., Tissue Eng Part A. 2009 Dec;15(12):3719-27
18. Bir SC, et al., J Vasc Surg 2009; 50(4):870-879.
19. Bir SC, et al., Circ J. 2008; 72(4):633-40
20. Bir SC, et al., J Vasc Res. 2011; 48(3): 195-205
21. Nagae M, et al., Tissue Eng. 2007; 13(1):147-58
22. Saito M, et al., Clin Exp Rheumatol. 2009; 27(2): 201-7
23. Ishida K, et al., Tissue Eng. 2007; 13(5):1103-12
24. Hokugo A, et al., Oral Surg Oral Med Oral Pathol Oral Radiol Endod. 2007 Jul; 104(1):44-8.
25. Michlits W, et al., Wound Repair Regen. 2007;15(3):360-367.
26. J.M. Stevenson, et al., Methods in Molecular Medicine, 1(78), Wound Healing, I:95-105.

## Claims

1. A lyophilized gelatin membrane containing an effective amount of at least one blood derivative selected from the group of: platelet rich plasma (PRP), platelet lysate (PL) platelet poor plasma (PPP), cryoprecipitate (CRYO), platelet membrane rich plasma (PMRP), for use as a tissue regenerating agent by topical administration.

2. The lyophilized gelatin membrane according to claim 1 wherein the effective amount of the at least one blood derivative is loaded onto the membrane prior to the lyophilization.

3. The lyophilized gelatin membrane according to claim 2 for a fast release of the at least one blood derivative.

4. The lyophilized gelatin membrane according to claim 1 obtainable by:
a) dissolving a gelatin powder in an effective amount at least one blood derivative to get a solution of gelatin / at least one blood derivative;
b) gelifying said solution to get a membrane of at least one blood derivative;
c) lyophilizing said membrane.

5. The lyophilized gelatin membrane according to claim 4 for a prolonged release of the at least one blood derivative.

6. The lyophilized gelatin membrane according to any of previous claims wherein the tissue is skin, derma and/or connective tissue.

7. The lyophilized gelatin membrane according to any of previous claims wherein the use as a tissue regenerating agent is due to a necrosis or a burning event.

8. The lyophilized gelatin membrane according to any of previous claims for wound or bed sores healing.

9. The lyophilized gelatin membrane according to any of previous claims wherein the use as a tissue regenerating agent is due to a metabolic or degenerative disease.
